# EUROPEAN PATENT APPLICATION

(11) **EP 3 695 848 A1**
(43) Date of publication of application: **19.08.2020**
(21) Application number: 18858431.2
(22) Date of filing: 13.09.2018
(51) Int. Cl.: A61K 36/85, A61K 36/185, A23L 33/105, A61P 3/00, A61P 3/04

(54) **COMPOSITION FOR CONTROLLING WEIGHT BY MODULATING LEVELS OF PEPTIDES INVOLVED IN FULLNESS AND/OR APPETITE**

(30) Priority: 25.09.2017 ES 201731147
(71) Applicant: Monteloeder, S.L., 03203 Elche (Alicante) (ES)
(72) Inventor: HERRANZ LÓPEZ, María, 03203 Elche (Alicante) (ES); ROCHE COLLADO, Enrique, 03203 Elche (Alicante) (ES); MICOL MOLINA, Vicente, 03203 Elche (Alicante) (ES); JONES BARBERÁ, Jonathan Richard, 03203 Elche (Alicante) (ES); CATURLA CERNUDA, Nuria, 03203 Elche (Alicante) (ES)
(74) Representative: Martin Alvarez, Juan Enrique
(86) International application number: PCT/ES2018/070600
(87) International publication number: WO 2019/058011

(57) **Abstract**

The present invention provides compositions which comprise an extract with at least 5 % in weight of anthocyanins and an extract with at least 15 % in weight of phenylpropanoids which are specially indicated for their use in weight reduction periods because they reduce appetite and increase satiety though digestive tract-secreted hormones level modification. In a parallel and complementary way, these compositions act on the lipid metabolism of the adipose tissue cells by activating AMPK sensors and the fat oxidative and elimination pathways. Said composition is obtained by hydro-alcoholic extraction of Rosella flowers and Lemon Verbena leafs, using in the case of Lemon Verbena alcohol concentrations (ethanol, methanol, propanol, isopropanol or butanol) greater than 70 and up to 100 % in volume).

## Description

### TECHNICAL FIELD OF THE INVENTION

The present invention is framed within the following technical fields: nutritional supplements, functional foods, pharmaceutical composition, and animal use; in order to control body weight and appetite, in particular, for use by overweight or obese people. In particular, it refers to a composition that comprises two polyphenolic plant extracts.

### BACKGROUND TO THE INVENTION

Obesity has been qualified by the World Health Organisation (WHO) as a global epidemic associated with several metabolic imbalances, which cause an increase in adipose tissue mass, endothelium dysfunction, dyslipidaemia, hypertension, atherosclerosis and insulin resistance. Overall, these imbalances constitute a complex pathology globally known with the term metabolic syndrome.

Obesity is defined as a condition of excess fat build-up in the adipose tissue, which implies a health risk and predisposes to insulin resistance, hypertension and dyslipidaemia. It is generally accepted that the only option to prevent the development of obesity related conditions is to restrict calories intake and to increase physical activity. However, these lifestyle changes are not feasible due to the fact that ageing is associated with a muscle mass loss and a reduction of metabolic expenditure at rest.

Obesity is generally treated with a combination of methods, which include adopting a healthy diet, exercise and psychological support in order to obtain tangible targets in weight reduction and efficient metabolic control of this pathology. However, these methods are difficult to implement among the overweight population in the long term, because individuals under treatment tend to quit and recover the lost weight. Furthermore, a weight loss period is accompanied by a body adaptation to the new metabolic requirements. An energy imbalance is produced between caloric intake and expenditure, which stimulates ghrelin secretion to induce appetite and cause a greater food intake, at the same time that the metabolic rate at rest is reduced and the energetic efficiency is increased (reduced energy expenditure in periods of activity). In the long term, this implies that the body tends to spend less calories and considerably increases its appetite, resulting in the known "weight bounces", in which the same or more food than before the diet is consumed and the body cannot burn the calories at the same rate as before the diet, with the consequent lost weight, or even a bigger weight, uptake. It is, therefore, necessary to modulate the metabolism to avoid increasing the appetite in diet and weight loss periods and to prevent or reduce comebacks in obesity or overweight treatments.

Obesity is a very complex pathology in which several factors intervene, such as a low intensity and chronic inflammatory component, an altered energetic metabolism and an oxidative imbalance. This complexity is part of the reason why weight and metabolic readjustments are very slow processes. In many instances, this implies a resource to complementary methods in order to achieve the desired weight reduction, such as bariatric surgery, pharmacological treatments and nutritional intervention with dietetic supplements or nutraceuticals.

Bariatric surgery has the same drawbacks as any surgical intervention, there being even cases of death during the procedure. The drawback of pharmacological treatments are well known too as "secondary effects", which can happen in more or less severity. Besides, there are many dietetic supplements or nutraceutical products of which their weight loss, fat burning or appetite reduction effects are widely advocated but which are not scientifically proven and which are doubtful, or in the best case, are the result of a placebo or autosuggestion effect.

### SUMMARY OF THE INVENTION

A first aspect of the invention provides a composition for use in an individual's food intake modulation by appetite reduction and/or satiety increase through the individual's adipose tissue or digestive tract secreted appetite-related peptides levels modification, characterised in that said composition comprises an extract with at least 5% in weight of anthocyanins and an extract with at least 15% in weight of phenylpropanoids.

A composition that contains both previously mentioned extracts is hugely effective in the food intake modulation because it causes satiating and appetite reducing effects, through its effect in the gut secreted peptide levels, which cause satiety or appetite feelings. With said composition it is possible to reduce the negative effects of weight control or maintenance strategies; for example, it is possible to reduce the appetite during a weight reduction period and thus, reduce or prevent the possibilities that the dieting individual starts ingesting food in greater than the recommended amounts and starts to gain weight back. Likewise, this composition is very useful in weight maintenance strategies, once the desired weight has been achieved, this composition helps to keep a satiety feeling for longer, and thus, the modulation of the food intake at a desired level can be achieved, for example, to maintain the body weight (or reduce it further, if necessary).

Preferably the extract with at least 5 % in weight in anthocyanins contains around 10 % in weight in anthocyanins.

Preferably the extract with at least 15 % in phenylpropanoids contains around 30 % in weight in phenylpropanoids.

These concentrations in anthocyanins and phenylpropanoids assure that the composition used is not cytotoxic.

Preferably the extract with anthocyanins comprises a Rosella (*Hibiscus sabdariffa*) extract.

Preferably the extract with phenylpropanoids comprises a Lemon Verbena (*Lippia citriodora*) extract.

The effects of Lemon Verbena and Rosella on appetite are not clearly defined in the state of the art. There are sources that consider that they have appetite increasing and satiety reduction effects, and other sources consider that they reduce the appetite and increase satiety. In the present description it will be proven that a composition with both extracts exhibits a potent effect in modifying the expression of peptides involved in the food intake (appetite and satiety), for example, ghrelin and leptin, and which, in comparison with equivalent separate doses of these extracts, produce a very significantly greater effect. Given the ambiguous effects of both extracts taken separately, it is highly surprising and unexpected the satiating and appetite reducing effect obtained with the compositions that contain both extracts.

Rosella is an anthocyanin-rich plant and its extract, combined with the Lemon Verbena extract, which is a phenylpropanoid-rich plant, produces a marked satiating and appetite reducing effect.

The composition may comprise a minimum of 50 % in weight of Lemon Verbena (*Lippia citriodora*) extract and a minimum of 25 % in weight of Rosella (*Hibiscus sabdariffa*) extract.

Preferably the composition comprises around 65 % in weight of Lemon Verbena (*Lippia citriodora*) extract and around 35 % in weight of Rosella (*Hibiscus sabdariffa*) extract.

This percentage of both extracts causes that the composition exhibits a greater effect on the fat metabolism activation mediated by a greater AMPK activation in comparison with a composition in which both extracts are mixed in equal amounts.

The composition may be administered in a dose of 250 to 1000 milligrams a day.

Preferably the composition is administered in a dose of 500 milligrams a day.

These doses ensure an effect on the appetite related peptides secreted by the digestive tract and adipose tissue of the individual, which translates in appetite reduction and/or satiety increase which is reflected in a lowered food intake need.

A second aspect of the invention provides a composition for use in an individual's weight reduction by lipid metabolism modification mediated by the individual's AMP activated protein kinase energetic (AMPK) sensors which translates into the individual's body fat percentage reduction, characterised in that said composition comprises an extract with at least 5% in weight of anthocyanins and an extract with at least 15% in weight of phenylpropanoids.

A composition that contains both previously mentioned extracts is hugely effective in an individual's weight reduction through a change or enhancement in the lipid metabolism through the activation of the AMP activated protein kinase energetic sensors (AMPK). This composition is very useful for treating obesity, because it is able to reduce the adipose tissue amount in an individual and it is able to improve other parameters typically related with being obese or overweight.

AMPK is a serine/threonine kinase which plays a very important role in keeping cell homeostasis. Its activation implies the activation of catabolic pathways such as lipolysis and fatty acids oxidation and the inhibition of anabolic pathways such as lipogenesis and glycogenesis.

Preferably the extract with at least 5 % in weight in anthocyanins contains around 10 % in weight in anthocyanins.

Preferably the extract with at least 15 % in phenylpropanoids contains around 30 % in weight in phenylpropanoids.

These concentrations in anthocyanins and phenylpropanoids assure that the composition used is not cytotoxic.

Preferably the extract with anthocyanins comprises a Rosella (*Hibiscus sabdariffa*) extract.

Preferably the extract with phenylpropanoids comprises a Lemon Verbena (*Lippia citriodora*) extract.

It has been proven that Rosella anthocyanins are particularly effective in reducing inflammation linked to metabolic stress by leptin and monocyte-1 chemoattractant protein (MCP-1) secretion inhibition. These are important adipokines which regulate the non-resident macrophage migration in adipose tissue and general systemic inflammation. The polyphenolic Rosella extracts also prevent hepatic steatosis in hyperlipidaemic mice thought the expression of the genes involved in glucose and lipid homeostasis. In high-fat fed mice, Rosella anthocyanins attenuate the blood glucose increase and the apparent increase in insulin resistance, and also cause the increase in breathing-rate, which is closely related with the basal metabolic rate (at rest) (BMR). In this same animal model, it was also observed a reduction in the expression of lipogenic genes, such as the gene encoding fatty acid synthases (FASN) and the sterol regulating element ligand protein (Srebp-1c), with a simultaneous hepatic AMPK activation.

It is known that the hepatic AMPK activation induces the expression of gen PPARGC1A and directly enhances its activity by phosphorylation, which in turn increases biogenesis and mitochondrial function. This implies that Rosella anthocyanins act upon fat usage, most probably by lipogenesis inhibition, and upon lipolysis activation at mitochondrial biogenesis level. All these processes resemble a metabolic situation which reflects an increased cellular energetic demand, an enhanced energetic expenditure and an increased basal metabolic rate.

On another hand, Lemon Verbena extract phenylpropanoids and its major component, verbascoside, enhance the metabolic alterations induced by a raised glucose level. These effects are mediated by a PPARy dependent transcriptional adiponectin upregulation and a potent AMPK activation, an RNA expressed PPAR-α upregulation and a FASN downregulation. Experiments carried out in mice indicate a fat metabolism enhancement (cholesterol and triglycerides), especially in triglycerides elimination.

This indicates a complementary effect unknown up to now from both extracts in the lipids metabolism and the AMPK activation. This implies that a composition as previously described has an unexpected and very significant effect in obese or overweight people's weight reduction, as explained in the examples.

The composition may comprise a minimum of 50 % in weight of Lemon Verbena (*Lippia citriodora*) extract and a minimum of 25 % in weight of Rosella (*Hibiscus sabdariffa*) extract.

Preferably the composition comprises around 65 % in weight of Lemon Verbena (*Lippia citriodora*) extract and around 35 % in weight of Rosella (*Hibiscus sabdariffa*) extract.

This percentage of both extracts causes that the composition exhibits a greater effect on the lipids metabolism activation mediated by a greater AMPK activation in comparison with a composition in which both extracts are mixed in equal amounts.

The composition may be administered in a dose of 250 to 1000 milligrams a day.

Preferably the composition is administered in a dose of 500 milligrams a day.

These doses ensure a significant effect on AMPK activation, which translates in a lipid metabolism activation and, as a consequence, a reduction in body fat percentage.

In more particular embodiments, the administration of the composition of the present invention is selected among parenteral, transdermal, oral, topical intracolonic or vaginal ways.

In a yet more particular embodiment, the composition of the present invention is administered in a parenteral way combined with conventional injectable liquid adjuvants, such as water or appropriate alcohols. The conventional pharmaceutical adjuvants for injection, such as stabilising agents, solubilising agents and buffers may be included in such injectable compositions. In a yet more particular embodiment, the composition of the present invention is administered intramuscularly, intraperitoneally or intravenously.

In a yet more particular embodiment, the composition of the present invention is administered orally, and contains one or more physiologically compatible excipients or vehicles, in liquid or solid form. These compositions may contain conventional components such as physiologically acceptable binder, filler, lubricant and humectant agents. The compositions can take any suitable shape, such as tablets, dragees, capsules, lozenges, oily or watery solutions, suspensions, emulsions or in dry powder form suitable for its reconstitution in water or other suitable liquid medium before use, for an immediate or controlled dosage.

In yet more particular embodiments of this invention, the liquid oral forms may also contain additives such as sweetening, flavouring, preservative and emulsifying agents. The non-aqueous compositions for oral administration may also be formulated containing, for example, edible oils. Such liquid compositions may be encapsulated is a convenient way in, for example, soft gelatine capsules in a unitary dose amount.

In yet more particular embodiments of this invention, the dosage of the pharmaceutical composition of the present invention is on a daily basis for humans and animals and may vary depending on the age, weight or degree of illness, etc. The daily dose for mammals, including human beings, normally varies from 250 milligrams until 1000 milligrams, preferably 500 mg of substance which is going to be administered during one or more ingestions.

A third aspect of the invention provides a method of production of a composition which comprises an extract with at least 5 % in weight of anthocyanins and an extract with at least 15 % in weight of phenylpropanoids, characterised in that it comprises a hydroalcoholic extraction of Lemon Verbena (*Lippia citriodora*) and an hydroalcoholic extraction of Rosella (*Hibiscus sabdariffa*).

This method uses two natural sources rich in the active compounds of the composition. The hydroalcoholic extraction of these plants allows obtaining the required active compounds content with notable yield and economic feasibility.

The lemon Verbena hydroalcoholic extraction may be carried out using a aqueous alcohol solution with an alcohol content greater than 70 % and up to 100% in volume of ethanol during two hours at a temperature between 40 y 80 °C.

Preferably, the ethanol in water solution contains around 70 % in volume of alcohol.

This composition of the extraction agent and the extraction conditions maximise the phenylpropanoid compounds obtention, in particular, verbascosides in Lemon verbena leafs in economically feasible conditions without having to distil an excess of water or alcohol.

### BRIEF DESCRIPTION OF THE DRAWINGS

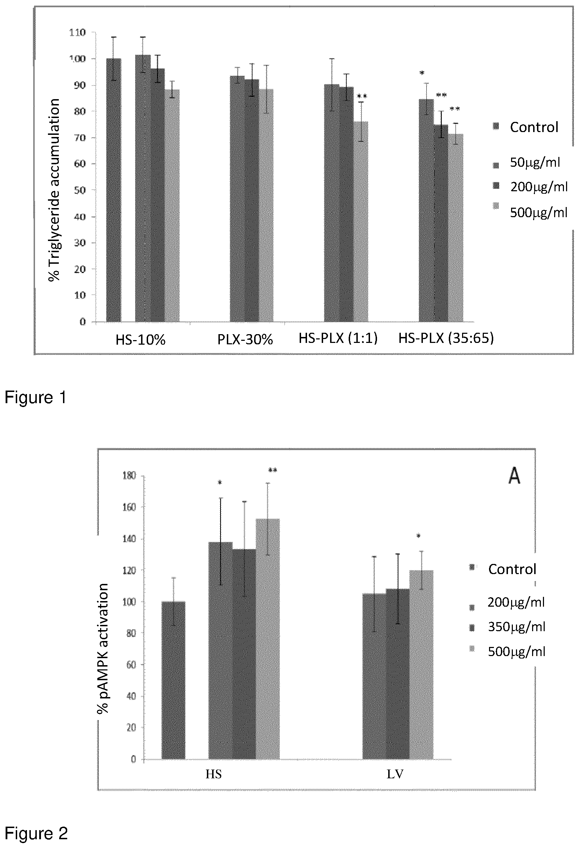

Figure 1. Graphic showing the percentage of triglyceride build-up in insulin-resistant hypertrophic adipocytes.
Figure 2. Graphic showing the AMPK activation percentage in insulin-resistant hypertrophic adipocytes treated with different Rosella and Lemon Verbena extract concentrations.

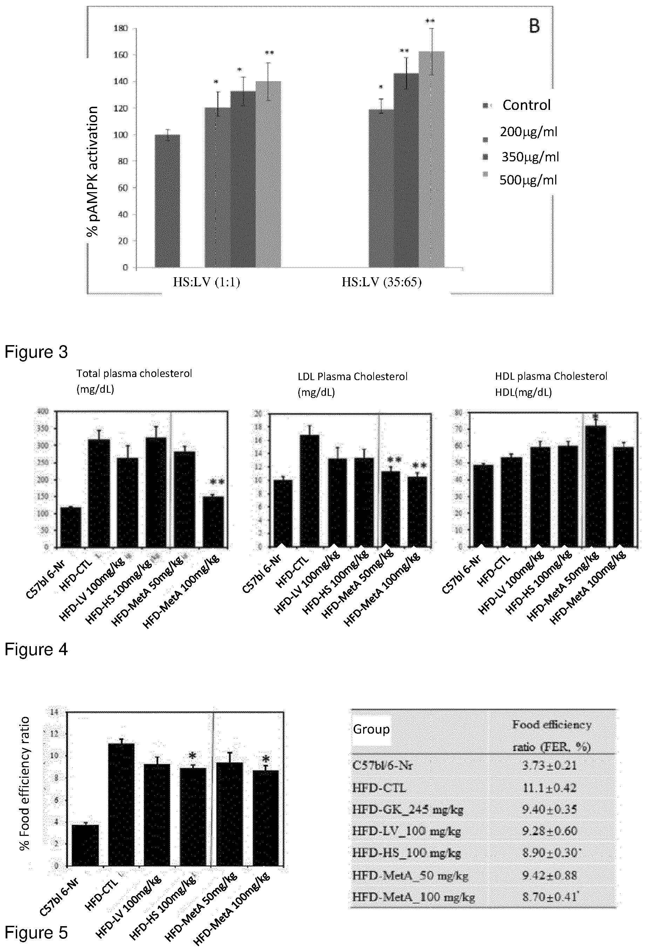

Figure 3. Graphic showing the AMPK activation percentage in insulin-resistant hypertrophic adipocytes treated with different concentrations of Lemon Verbena and Rosella extracts mixtures, with two different ratios.
Figure 4. Graphics showing total cholesterol, LDL cholesterol (bad) and HDL cholesterol (good) levels in blood of high-fat diet fed mice and treated separately with Lemon Verbena and Rosella extract, and treated with two different doses of a mixture of Lemon Verbena and Rosella extracts in a 65:35 weight ratio, respectively, and of non-treated high-fat diet fed mice and of normal diet fed mice.
Figure 5. Graphic and table showing food efficiency ratio in high-fat diet fed mice and treated separately with Lemon Verbena and Rosella extract, and treated with two different doses of a mixture of Lemon Verbena and Rosella extracts, respectively, and of non-treated high-fat diet fed mice and of normal diet fed mice.

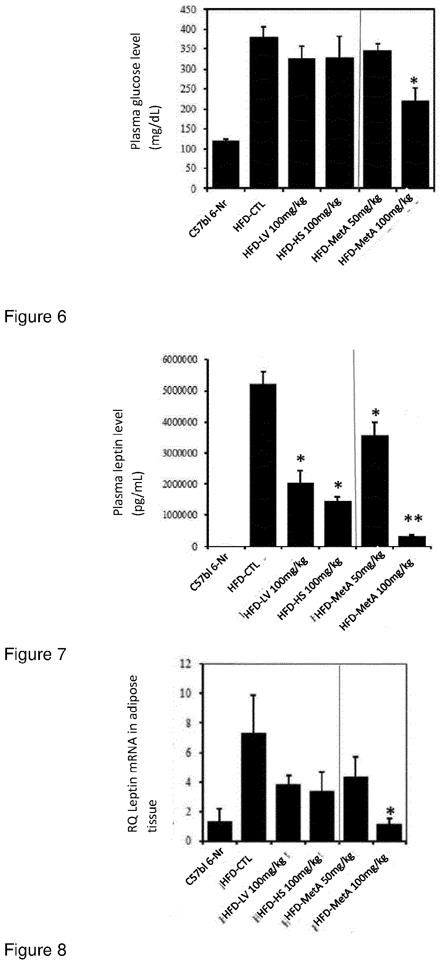

Figure 6. Graphic showing blood glucose levels in high-fat diet fed mice and treated separately with Lemon Verbena and Rosella extract, and treated with two different doses of a mixture of Lemon Verbena and Rosella extracts, respectively, and of non-treated high-fat diet fed mice and of normal diet fed mice.
Figure 7. Graphic showing blood leptin levels in high-fat diet fed mice and treated separately with Lemon Verbena and Rosella extract, and treated with two different doses of a mixture of Lemon Verbena and Rosella extracts, respectively, and of non-treated high-fat diet fed mice and of normal diet fed mice.
Figure 8. Graphic showing adipose tissue leptin levels in high-fat diet fed mice and treated separately with Lemon Verbena and Rosella extract, and treated with two different doses of a mixture of Lemon Verbena and Rosella extracts, respectively, and of non-treated high-fat diet fed mice and of normal diet fed mice.

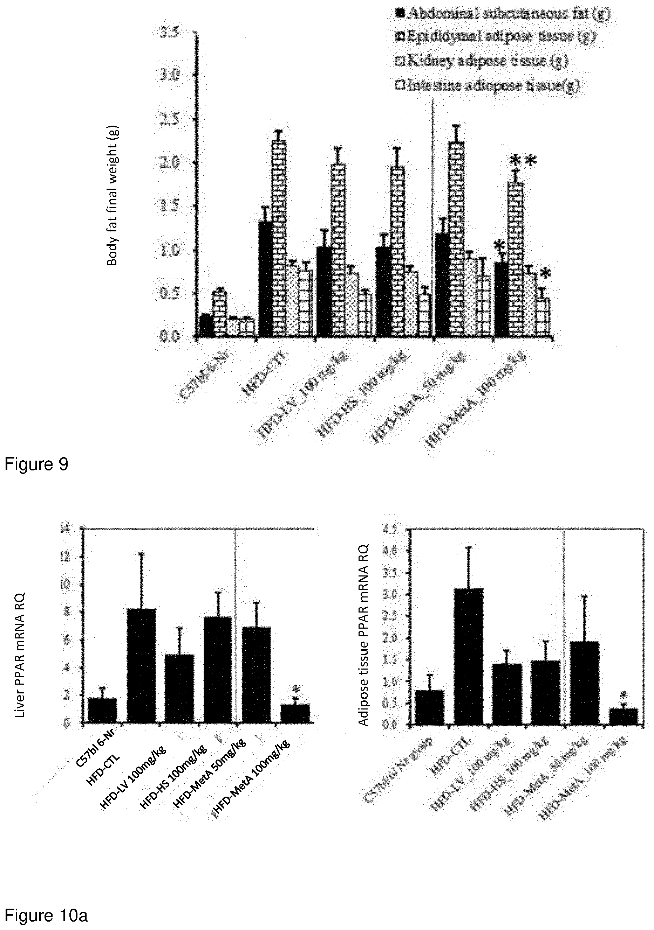

Figure 9. Graphic showing body fat weight in high-fat diet fed mice and treated separately with Lemon Verbena and Rosella extract, and treated with two different doses of a mixture of Lemon Verbena and Rosella extracts, respectively, and of non-treated high-fat diet fed mice and of normal diet fed mice.

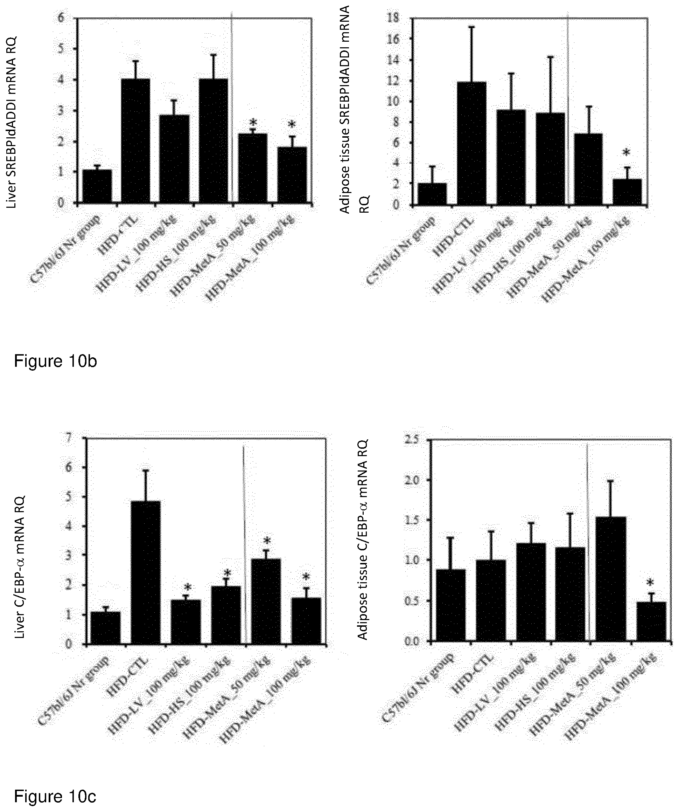

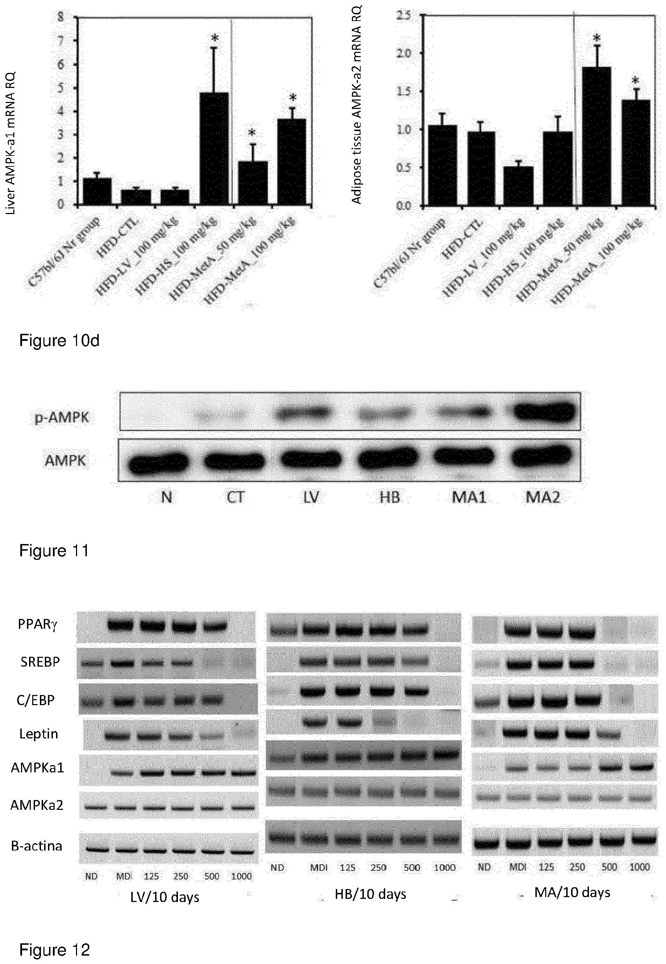

Figures 10a, 10b, 10c and 10d. Graphics showing abdominal adipose tissue and liver metabolic markers in high-fat diet fed mice and treated separately with Lemon Verbena and Rosella extract, and treated with two different doses of a mixture of Lemon Verbena and Rosella extracts, respectively, and of non-treated high-fat diet fed mice and of normal diet fed mice.
Figure 11. Western Blot analysis graphic carried out to determine protein amount in adipose tissue of high-fat diet fed mice and treated separately with Lemon Verbena and Rosella extract, and treated with two different doses of a mixture of Lemon Verbena and Rosella extracts, respectively, and of non-treated high-fat diet fed mice and of normal diet fed mice.
Figure 12. Graphics showing the genes corresponding to metabolic markers of immortalised human adipocytes, non-differentiated (ND) and differentiated (MDI), cultured in-vitro and treated separately with different doses of Lemon Verbena (LV) and Rosella (HS) extracts, and with different doses of a Lemon Verbena and Rosella extracts mixture.

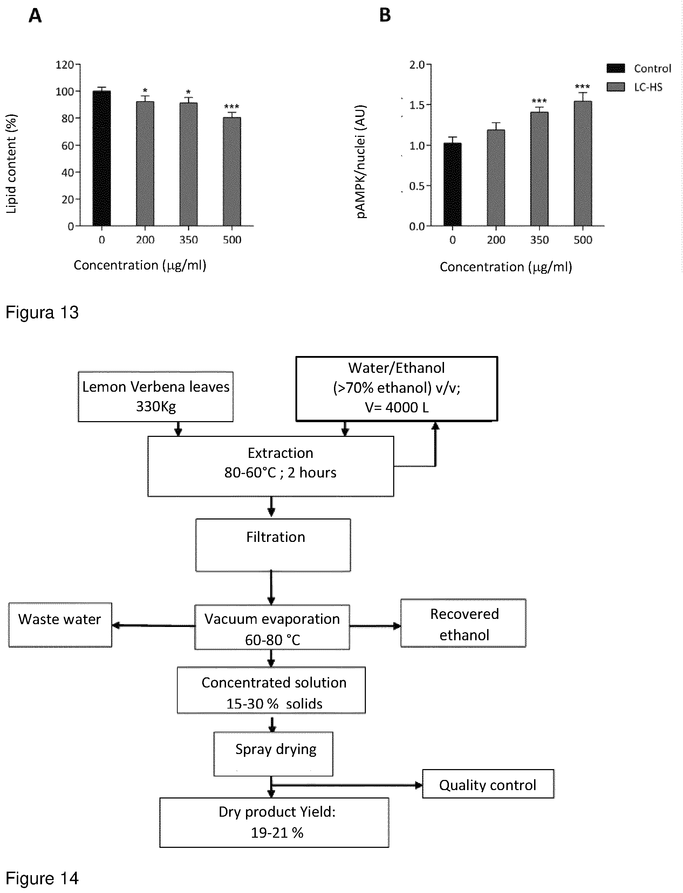

Figure 13. Graphics showing the lipid content decrease in hypertrophic adipocytes treated with the composition with Lemon Verbena and Rosella extracts and the parallel increase of the AMPK activation in said adipocytes.
Figure 14. Schematic drawing of the production method of a Lemon Verbena extract.

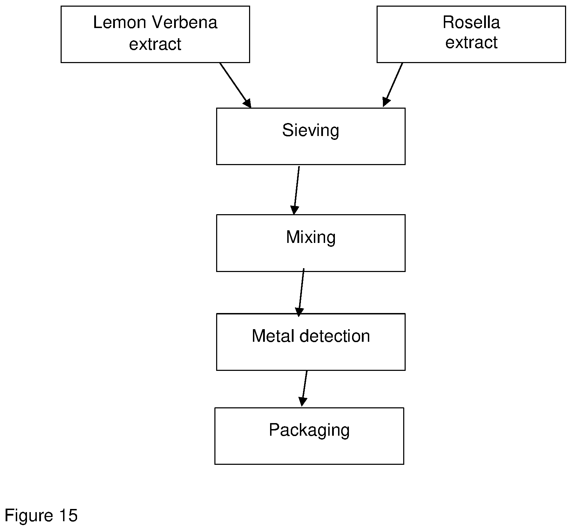

Figure 15. Schematic drawing of the production method of the composition according to an aspect of the present invention.

### DETAILED DESCRIPTION OF THE INVENTION AND EXAMPLES

### EXAMPLE 1. Lemon Verbena and Rosella extracts ratio determination.

3T3-L1 preadipocytes were cultured in 96-well plates with a complete culture medium (DMEM with low glucose at 1g/L) and sodium pyruvate, enriched with glutamine and supplemented with bovine serum at 10 % and antibiotics (100□g/mL streptomycin and 100 U/mL penicillin).

The adipocyte differentiation was induced by adding adipogenic agents (0.5 mM IBMX, 1 □M DEX and 1 □M insulin) to the culture medium during two days. The medium was replaced with fresh medium every 48 hours.

The phenotypic change in the adipocytes was observed with a microscope. In all experiments, more than 90 % of the cells were mature adipocytes after 8-10 days incubation. In order to induce cellular hypertrophy, the adipocytes were exposed to a high glucose medium (25 mM).

The cell cultures were carried out in sterile conditions using an air laminar flow hood for cell cultures and an incubator at 37 °C with a humidified atmosphere with 5 % in CO₂.

The extracts and the extracts composition effect on the triglycerides build-up and on the cytoplasmic AMPK activation in 3T3-L1 adipocytes was studied. The extracts were added after the adipocytes were differentiated until the plates were analysed. The extracts were previously prepared with culture medium and filtered with a de 0,2 µm filter in order to sterilise them.

The 29 days differentiated hypertrophic adipocytes were separately exposed to Rosella Extract (∼10 % weight in anthocyanins) and to Lemon Verbena extract (∼30 % weight in phenylpropanoids) and to 50:50 and 65:35 weight ratio mixtures of lemon Verbena to Rosella extracts. The extracts were added the 24^{th} day after adipocytes differentiation and were incubated at 50, 200 and 500 □g/mL during 5 days.

After these 5 days, the lipid content of the adipocytes was evaluated with the commercial reactant AdipoRed™ and using a multi-mode microplate reader with cellular image capture Cytation 3 from Biotek with a 485 nm excitation wavelength and a 572 nm emission wavelength. The lack of cytotoxicity was evaluated with the purple crystal method. The results are shown in Figure 1, with a graphic showing the percentage of triglycerides build-up in hypertrophic insulin-resistant adipocytes. HS represents the adipocytes treated with a 10 % anthocyanins Rosella extract, LV represents the adipocytes treated with a 30 % phenylpropanoids Lemon Verbena extract, HS-LV (1:1) represents the adipocytes treated with a 1 to 1 weight mixture of Lemon verbena and Rosella extracts and HS-LV (36:65) represents the adipocytes treated with a 35:65 in weight mixture of Rosella and Lemon Verbena extracts, respectively. P values are: *p<0,05, **p<0,01 y ***p<0,001.

AMPK and phosphorylated (activated) AMPK levels were determined with a quantitative immunofluorescence experiment using AMPK and phosphorylated AMPK antibodies. Moreover, the nuclei were marked with the reactant Hoechst 33342. Once the cells were marked, the AMPK fluorescence was quantified with a 490 nm excitation wavelength and a 520 nm emission wavelength, the phosphorylated AMPK fluorescence was quantified with a 593 nm excitation wavelength and a 614 nm emission wavelength and the nuclei fluorescence with a 350 nm excitation wavelength and with a 461 nm emission wavelength, using a multimodal plate reader.

The results are shown in Figures 2 and 3 which show graphics representing the AMPK activation percentage in hypertrophic insulin-resistant adipocytes treated with different doses of Rosella and Lemon Verbena extracts, separately, and combined in two different weight ratios. It can be seen that the most AMPK activation is produced with the greater dose of the composition which contains both extracts in a 65:35 weight ratio of Rosella extract to Lemon verbena extract, respectively. The p values are: *p<0,05, **p<0,01 y ***p<0,001, which indicate statistically significant differences with the control adipocytes (non-treated adipocytes). The legends have the same meaning as in Figure 1.

### EXAMPLE 2. Body fat, hyperlipidaemia, hyperglycaemia and adipogenesis reduction evaluation in obese mice treated with a composition which contains Lemon verbena and Rosella extracts.

*In-vivo* experiments were carried out with normal mice to which a high fat diet was fed (HFD) during 8 weeks. Total blood cholesterol, as well as HDL cholesterol (good cholesterol or high-density lipoprotein) and LDL cholesterol (bad cholesterol or low-density lipoprotein) were analysed.

The results are shown in Figure 4 wherein C57bl/6-Nr corresponds to normal diet mice, HFD-CTL to high-fat diet mice (HFD), HFD-LV to high-fat diet mice taking a -30 % weight phenylpropanoids Lemon Verbena extract, HFD-HS to high-fat diet mice taking a -10% weight anthocyanins Rosella extract, and HFD-MetA to high-fat diet mice taking a Lemon Verbena and Rosella extracts composition with 65:35 weight ratio, respectively.

The results show that mice taking both extracts composition had lower total cholesterol levels, coinciding with a decrease in LDL and an increase in HDL. These values were lower than with the extracts taken separately (* p<0,05, ** p < 0,01 with respect to the control HFD-CTL).

The food amount taken by mice was analysed daily, with respect to the animals' weight. This value is known as Food Efficiency Ratio. A lower amount implies a lower food intake with respect to its weight. The results can be seen in Figure 5. (* p<0,01 with respect to control HFD-CTL)

The blood glucose level was also analysed, given that the high-fat diet mice are hyperglycaemic. As it can be seen in Figure 6 ((* p<0,05 with respect to control HFD-CTL), mice taking the highest concentration of the extracts composition were the only ones with an important blood glucose level reduction, close to the normal diet mice.

Leptin hormone level was analysed in mice taking a high fat diet. Specifically, the blood level of such hormones was analysed, as it can be seen in Figure 7 (*p<0,05, **p<0,01 with respect to control HFD-CTL).

Comparing the control with the normal diet (C57bl/6J Nr) with respect to the high fat diet mice (HFD-CTL), it can be seen that leptin has a significantly higher expression in the high-fat diet mice. Leptin induces satiety, however, it is known that the membrane receptors of said hormone have an high resistance in obese individuals, and require high leptin levels in order to be stimulated and therefore to activate the signal cascade to induce satiety. Consequently, detecting high leptin levels in high-fat diet mice, consequently obese, confirms the resistance to this hormone.

The intake of any of the tested extracts or mixtures (Lemon Verbena, Rosella or mixture thereof) decreases the leptin expression in blood, even though only in the case of the extracts mixture composition we can see similar levels to normal diet mice, i.e., non-obese. Therefore, this mixture composition intake can reverse the leptin overexpression, given that it eliminates the resistance to said hormone, thus allowing that HFD mice have satiety levels similar to normal diet mice.

These data coincide the FER observed results, wherein mice that took the both extracts composition took less food amounts with respect to their weight.

A similar result can be seen in Figure 8 with regards to the leptin expression in adipose tissue, wherein it can be seen that only in mice taking the extracts composition the leptin expression in adipose tissue decreased (*p<0,05 vs HFD-CTL), achieving levels observed in normal diet mice. This is due to a possible leptin receptors sensitisation in mice that took the extract composition, such that a lower leptin blood concentration was enough to cause satiety feelings.

The combined and separate effect of Lemon Verbena and Rosella extracts on the adipose tissue metabolism has been studied. In the first place, the amount of adipose tissue of the mice was determined, isolating and weighting the abdominal, epididymis, kidney and intestinal fat.

As it can be seen in Figure 9, only mice that took the extracts composition presented significantly less fat amount in the abdomen, epididymis and intestine (*p<0,05, **p<0,01). These values agree with the previously commented results, i.e., that these mice take less food, by means of satiety regulation, which results in weights loss by means of adipose tissue loss.

Besides the weight, adipose tissue and liver metabolic markers were studied (in the case of overweight/obesity there is an abnormal build-up of adipose tissue in the liver, known as "fatty liver") in relation with the accumulation or regulation of triglycerides and fatty acids. The markers analysed by means of RT-PCR were PPARy, SREBP1 (ADD1), C/EBPa and AMPK.

PPARγ is a nuclear membrane receptor found in adipocytes, as well as in colonic cells and macrophages. In the case of adipose tissue, their function is to activate lipogenesis and lipid catchment. SREBP1 c regulates lipids homeostasis, and its activation induces cholesterol synthesis when this is low. C/EBPa is a transcription factor that regulates adipogenesis as well as normal adipocytes function, and regulates the PPARγ expression. Finally, AMPK (AMP kinase) inhibits lipids catchment, cholesterol synthesis and lipogenesis, among other functions, though in order to carry out its function, the protein must be phosphorylated (p-AMPK).

As it can be appreciated in the Figures 10a to 10d histograms, the PPARγ y SREBP1 c (ADD1) expression significantly decreases only in mice that took the extracts composition, studied in the liver as well as in the abdominal adipose tissue (*p<0,05). Regarding C/EBPa, it is also seen an expression decreasing effect with Lemon Verbena and Rosella, but only in the liver, whereas the extracts composition decreased this factor expression in both analysed tissues. In these three genes, the resulting expression in mice taking the extracts composition was similar to that obtained in control mice taking a normal diet.

On another hand, the AMPK transcriptional expression was increased in mice taking the extracts compositions (in the liver, mice taking Rosella also exhibited an expression increase, but not in the adipose tissue).

This result was also confirmed by means of a Western Blot analysis, which allows the analysis of the protein amount present in the adipose tissue and which results are shown in Figure 11. The legend is interpreted as: N mice with normal diet, CT non-treated mice with high-fat diet, LV Lemon Verbena extract treated mice with high-fat diet, HS Rosella extract treated mice with high-fat diet, MA1 high-fat diet mice treated 65:35 weight ratio composition of Lemon verbena and Rosella extracts at a 50 mg/kg dose and MA2 high-fat diet mice treated 65:35 weight ratio composition of Lemon verbena and Rosella extracts at a 100 mg/kg dose.

The active form of AMPK is phosphorylated AMPK (p-AMPK). As it can be seen in the Figure, the adipose tissue of mice taking the extract composition were those which exhibited more p-AMPK concentration in active protein form.

Therefore, mice taking a high-fat diet for 8 weeks (HFD-CTL) show activation of genes involved in fat accumulation, lipogenesis, triglycerides and cholesterol synthesis, adipogenesis, which results in a greater content in body fat, compared with control mice with a normal diet (C57bl/6J Nr). However, when these mice take the extracts composition, these genes expression is reverted to concentrations similar to the ones observed in non-obese normal diet mice. Moreover, the p-AMPK activation indicates that the fatty acids metabolism is increased, thus decreasing its storage at the same time as its degradation increases.

These *in-vivo* results carried out on mice were confirmed with *in-vitro* studies using a human adipocytes cellular line, wherein several concentrations of the different ingredients were tested and incubated for 10 days. The same genes as the ones studied in mice were analysed for the fatty acid metabolism. The results are shown in Figure 12. (ND: non-differentiated cell cultures, MDI: differentiated cell cultures, LV: Lemon Verbena, HB: Rosella, MA: LV and HB extracts composition, 125-1000: ingredients concentrations expressed in µg/ml)

In the case of PPARγ, SREBP1c(ADD1), C/EBPa, a decrease of their expression is observed in the three studied cases with respect of the differentiated adipocytes (MDI), specifically in the 1 mg/ml concentration of Lemon Verbena and Rosella, and also in the 0.5 as well as in the 1 mg/ml concentration of the extracts composition. Therefore, the extracts composition can be used at lower concentrations to induce a repressing effect of said transcripts, compared with the individual extracts.

Regarding AMPK, it can be observed an important expression increase in the case of the extracts composition at 0.5 and 1 mg/ml, whereas with the separate extracts, no statistically significant differences can be observed with respect to the differentiated cell culture (MDI).

### EXAMPLE 3. Appetite reduction and/or satiety increase by means of changes in digestive tract and adipose tissue-secreted peptide levels due to intake of a composition that contains a Lemon verbena extract with a 30% weight content in phenylpropanoids and a Rosella extract with a 10 % weight content in anthocyanins, in a 65:35 weight ratio.

54 women, aged between 35 to 75 years and with a body mass index between 25 to 35 kg/m²were selected. The study is a random double-blind, placebo-controlled assay of 8 weeks carried out at the Miguel Hernandez University of Elche, in Spain. After recruitment, the volunteers were randomly assigned to a placebo control group L2 (n=26) or to an experimental group L1 (n= 28).

L1 group members (mean age 51 years) received a 500 mg daily dose of a composition that contains Lemon Verbena and Rosella extracts.

L2 group members (mean age 51 years) received a 500 mg daily dose of microcrystalline cellulose.

The placebo and the satiety effect composition capsules had the same size, colour, smell and weight. The volunteers took a capsule 20 to 30 minutes before breakfast every day for two months.

The volunteers were required to follow a balanced isocaloric diet and to walk at least 30 minutes a day.

The fulfilment of these requirements was monitored during the clinical visits or by telephonic interviews each week. A total of 3 and 4 participants were excluded from L2 and L1 groups, respectively.

Several parameters were monitored during the study. Anthropometric measurements were taken at the start of the study, after one months and after two months from the start of the study, and include body weight, height, triceps, biceps and abdominal skinfold measurements and arm and abdominal perimetric measurements. The abdomen perimeter (AC) was measured in two different places: frontally midways between the xiphoid process and the belly button and laterally midways between the thoracic chest end and the iliac crests (AC1) and at the belly button (AC2). The body fat percentage was obtained from the abdominal measurement using the Weltman equation. Moreover, the heart rate at rest and diastolic and systolic tension were measured at the start, and after one and two months of study.

The validated analogue visual scale test (VAS test) was used to register hunger, satiety, fullness feeling, prospective food intake, desire to eat something fatty, savoury, sweet or no sweet and the flavour appreciation of the meals. The VAS test was completed at the start of the study at rest, after 15, 20, 45 and 60 days of study. In a complementary way to the subjective evaluation of their heath state, the volunteers filled a SF-36 questionnaire at the start and at the end of the study. Finally, blood samples were obtained from the forearm vein after overnight fasting at the start, and after 30 and 60 of study. The plasma was immediately processed and the samples were stored at - 80 Celsius degrees.

Table 1 shows the anthropometric parameters at the start and during the study. Statistically significant differences between the two groups cannot be seen at the start of the study. Table 1 results indicate a statistically significant improvements of the anthropometric parameters after two months study in the L1 group, which took the Lemons Verbena and Rosella extracts composition, specially improving the body fat percentage, the triceps skinfold, body weight and the hip perimeter (AC2). These differences can be more clearly seen in Table 1a.

**Table 1. Changes in the anthropometric parameters during the study expressed in mean values plus/minus standard deviations. The intra-group statistical analysis was determined in each period in comparison with the data at the start of the study. The significance was established at: *p<0,05, **p<0,01, ***p<0,001, ****p<0,0001.**

| | **L2 Group** | | | **L1 Group** | | |
|---|---|---|---|---|---|---|
| **Anthropometric parameters** | **Start** | **Month 1** | **Month 2** | **Start** | **Month 1** | **Month 2** |
| Body weight (kg) | 75.64 ± 12.92 | 74.20 ± 12.62**** | 73.56 ± 12.57**** | 75.26 ± 9.06 | 72.80± 9.45**** | 71.78 ± 9.06**** |
| Body mass index (kg/m2) | 29.78 ± 4.19 | 29.15 ± 4.08**** | 28.95 ± 4.01**** | 29.60 ± 3.40 | 28.60 ± 3.52**** | 28.26 ± 3.46**** |
| Arm perimeter (cm) | 31.20 ± 3.80 | 31.23 ± 3.80 | 30.98 ± 4.10 | 30.58 ± 1.67 | 30.47 ± 1.83 | 30.26 ± 1.76**** |
| AC1 (cm) | 94.02 ± 13.03 | 92.84 ± 12.86*** | 92.05 ± 13.16**** | 90.96 ± 9.03 | 88.96 ± 9.18**** | 88.01 ± 8.90**** |
| AC2 (cm) | 100.7 ± 14.01 | 100.5 ± 14.21 | 99.90 ± 14.54** | 96.42 ± 7.93 | 94.84 ± 7.87**** | 93.85 ± 7.95**** |
| Hip perimeter (cm) | 108.8 ± 8.73 | 108.15 ± 8.55** | 107.5 ± 8.48*** | 110.4 ± 7.23 | 108.2 ± 7.56**** | 106.9 ± 7.48**** |
| Triceps skin fold (mm) | 43.25 ± 9.28 | 43.15 ± 9.34 | 43.10 ± 9.42 | 41.62 ± 8.18 | 40.96 ± 8-08** | 39.98 ± 7.97**** |
| Biceps skin fold (mm) | 41.53±14.46 | 41.25±14.35* | 41.09±14.35* | 38.33±10.63 | 37.61±10.87* | 36.87±10.52**** |
| Abdominal skin fold (mm) | 35.72±11.15 | 35.39±10.68 | 34.39±10.68 | 41.45±12.89 | 39.72±12.95*** | 38.60±13.08*** |
| % body fat | 44.98 ± 2.71 | 44.69 ± 2.65*** | 44.53 ± 2.64**** | 44.66±2.04 | 44.10 ± 2.10**** | 43.83±2.06**** |

| **Vital signs** | | | | | | |
|---|---|---|---|---|---|---|
| Heart rate (bpm) | 71.41 ± 8.89 | 71.95 ± 8.85 | 72.09 ± 9.24 | 73.32 ± 9.70 | 70.84 ± 7.54* | 68.64 ± 7.07**** |
| Systolic tension (mmHg) | 114.5 ± 23.26 | 115.1 ± 23.55* | 115.2 ± 23.13* | 117.4 ± 12.13 | 115.1 ± 12.82** | 113.9 ± 12.5**** |
| Diastolic tension (mmHg) | 73.73 ± 10.57 | 74.05 ± 11.50 | 73.73 ± 10.70 | 73.40 ± 5.00 | 71.28 ± 5.89**** | 69.48 ± 6.62**** |

**Table 1a. Changes in the anthropometric parameters during the study expressed in mean values plus/minus standard deviations. The intra-group statistical analysis was determined in each period in comparison with the data at the start of the study. The significance was established at: *p<0,05, **p<0,01, ***p<0,001, ****p<0,0001.**

| | **Differences after 1 month** | | **Differences after 2 months** | |
|---|---|---|---|---|
| **Anthropometric parameters** | **Placebo (L2, n= 22)** | **MetabolAld® (L1, n= 25)** | **Placebo (L2, n= 22)** | **MetabolAld® (L1, n= 25)** |
| Body weight (kg) | -1.44 ± 0.27 | -2.46 ± 0.28* | -2.08 ± 0.30 | -3.48 ± 0.40* |
| Body mass index (kg/m2) | -0.63 ± 0.14 | -1.00 ± 0.15* | -0,83 ± 0.12 | -1,37 ± 0,16* |
| Arm perimeter (cm) | 0.03±0.03 | -0.11±0.06 | -0.22 ± 0.23 | -0.32 ± 0.08** |
| AC1 (cm) | 1.18±0.29 | -2.00±0.27* | -1.97 ± 0.37 | -2.95 ± 0.36 |
| AC2 (cm) | 0.20±0.54 | -1.58±0.25** | -0.80 ± 0.55 | -2.57 ± 0.34** |
| Hip perimeter (cm) | 0.65±0.23 | -2.20±0.32*** | -1.30 ± 0.28 | -3.50 ± 0.37**** |
| Triceps skin fold (mm) | -0.10 ± 0.16 | -0.66±0.18* | -0.15 ± 0.15 | -1.64 ± 0.29**** |
| Biceps skin fold (mm) | -0.28±0.14 | -0.62±0.22 | -0.44 ± 0.19 | -1.46 ± 0.29** |
| Abdominal skin fold (mm) | -0.33±0.36 | -1.73±0.37* | -1.33 ± 0.29 | -2.84 ± 0.53* |
| % body fat | 0.29±0.06 | -0.56±0.06** | -0.45 ± 0.07 | -0.83 ± 0.08*** |
| | | | | |
| Heart rate (bpm) | 0.54 ± 0.28 | -2.48 ± 0.89**** | 0.68 ± 0.36 | -4.68 ± 0.36**** |
| Systolic tension (mmHg) | 0.60 ± 0.28 | -2.30 ± 0.64**** | 0.70 ± 0.30 | -3.50 ± 0.61**** |
| Diastolic tension (mmHg) | 0.32 ± 0.43 | -2.12 ± 0.39**** | 0 ± 0.50 | -3.92 ± 0.63**** |

According the scores shown in Table 2, the Lemon Verbena and Rosella extracts composition consumption decreases the hunger feeling consistently along the two months of study. The mean hunger score decreased from 5.92 in day 15 to 2.58 in day 60 in group L1. Whereas in group L2 it increased from 6.18 in day 15 to 6.41 in day 60 in the placebo group. A similar trend can be observed in the scores to question 4.

Satiety increased from a 5.04 value in day 15 until a 7.58 value in day 60 in group L1, whereas it decreased from a 4.82 values at day 15 to a 4.22 value at day 60 in placebo group L2. A similar trend was observed in the scores to question 3.

**Tabla 2. Scores of appetite-related feelings in group L1 (extracts composition) and in group L2 (placebo) after 15, 30, 45 and 60 days of study, expressed as mean values plus/minus standard deviation. Data are expresses each question score in comparison with placebo. Statistically significant differences were established: *p,0.05, **p<0.01, ***p<0.001, ****p<0.0001. The scores range according to the following scheme:**

| | **15 days** | | **30 days** | | **45 days** | | **60 days** | |
|---|---|---|---|---|---|---|---|---|
| **Items** | **L1** | **L2** | **L1** | **L2** | **L1** | **L2** | **L1** | **L2** |
| 1. How hungry are you? | 5.92±2.48 | 6.18±2.36 | 4.00±2.34** | 6.05±1.73 | 3.29±2.37**** | 6.18±2.11 | 2.58±2.35**** | 6.41±1.87 |
| 2. How satisfied do you feel? | 5.04±2.07 | 4.82±1.89 | 5.92±2.02* | 4.32±1.95 | 6.45±2.32** | 4.63±1.50 | 7.58±1.72**** | 4.22±1.38 |
| 3. How full do you feel? | 4.65±1.94 | 4.45±1.76 | 5.76±1.80* | 4.30±1.90 | 6.42±1.87**** | 3.86±1.83 | 7.46+1.59**** | 3.52±1.91 |
| 4. How much do you think you can eat? | 5.57±2.83 | 7.22±1.52 | 4.14±1.98**** | 6.95±1.90 | 2.83±2.20**** | 6.82±1.74 | 2.54±1.90**** | 7.33±1.90 |
| 5. Would you like to eat something sweet? | 2.04±2.23 | 2.45±2.70 | 5.65±2.55** | 2.80±2.76 | 4.83±2.73*** | 1.95±2.13 | 6.67±2.18**** | 1.82±2.17 |
| 6. Would you like to eat something savoury? | 5.45±3.42 | 5.09±3.53 | 6.38±2.73**** | 2.60±1.79 | 7.57±2.27**** | 2.86±2.59 | 7.75±2.19**** | 3.09±2.72 |
| 7. Would you like to eat something not sweet? | 2.70±2.25* | 4.68±2.93 | 4.96±2.51 | 3.68±2.73 | 6.50±2.19**** | 2.90±2.75 | 7.67±1.40**** | 2.71±2.47 |
| 8. Would you like to eat something fatty? | 4.29±3.58* ** | 8.07±2.52 | 6.87±3.18 | 5.60±3.58 | 8.13+2.71** | 5.18±3.88 | 8.21±2.47** | 5.14±3.48 |

| | | | | | | | | |
|---|---|---|---|---|---|---|---|---|
| 1. 0 "Not at all hungry" - 10 "I have never been so hungry". 2. 0 "I feel completely empty" - 10 "I cannot take even a bite more" 3. 0 "I am not at all full" - 10 "I am completely full" 4. 0 "Nothing at all" - 10 "A huge amount" 5. 0 "Yes, a lot" - 10 "Not at all" 6. 0 "Yes, a lot" - 10- "Not at all" 7. 0 "Yes, a lot" - 10- "Not at all" 8. 0 "Yes, a lot" - 10- "Not at all" | | | | | | | | |

**Tabla 3. Appetite and satiety-related gut-secreted peptide levels in blood determined at the start and at the end of the study (second month), expressed as mean values plus/minus standard deviations. Significance was established for: *p<0,05.**

| | **Placebo (L2, n= 10)** | | **Composition with extracts (L1, n= 10)** | |
|---|---|---|---|---|
| | **START** | **MONTH 2** | **START** | **MONTH 2** |
| **GLP-1** (pg/mL) | 5,66 ± 1,27 | 4,228 ± 2,04* | 5,22 ± 1,43 | 6,82 ± 1,78* |
| **GHRELIN** (pg/mL) | 30,64 ± 0,64 | 33,74 ± 3,48* | 32,70 ± 4,82 | 32,89 ± 3,23 |
| **LEPTIN** (pg/mL) | 9669 ± 6617 | 5237 ± 4886 | 9772 ± 4455 | 4311 ± 2304* |
| **PYY** (pg/mL) | 7,84 ± 4,15 | 5,82 ± 5,01 | 9,80 ± 0,31 | 9,81 ± 0,24 |
| **RESISTIN** (pg/mL) | 15593 ± 6718 | 10799 ± 6624 | 18101 ± 6274 | 12049 ± 4022* |

It is necessary to mention that some analysed peptides (such as GLP-1, PYY and ghrelin) are secreted after food intake. Ghrelin stimulates appetite, whereas GLP-1 and PYY are secreted together to promote the satiety feeling. Given that the blood samples were taken before breakfast in the morning, the particular peptide levels correspond to basal levels. Initially, the basal levels in both groups match and no statistically significant differences between them are observed. The other peptides show statistically significant differences in group L1. In this context, the incretin GLP-1 significantly increased in group L1 whereas it decreased in group L2 and the ghrelin increased in group L2 and was kept constant in group L1. Therefore, group l1 volunteers had less appetite and more fullness feeling than the group L2 volunteers.

On another hand, leptin, which is synthesised in the adipose tissue and produces satiety feeling, significantly decreased in group L1 whereas no changes were observed in group L2. Resistin decreased significantly in group L1 whereas no changes were observed in group L2. The increased leptin level in the blood samples of group L2 is explained by the resistance in the corresponding receptors to cause satiety in response to leptin secretion, it is like the corresponding receptors were desensitised, whereas in group L1 the normal blood leptin level indicates that the corresponding receptors maintain the same or greater sensitivity to the leptin level.

Generally, these changes in the hormone (peptides) levels correlate positively with the questionnaire scores about appetite and satiety and with the anthropometric parameters evolution previously shown.

### EXAMPLE 4. Body weight reduction by means of lipid metabolism modification mediated by an AMP activated protein kinase energetic sensors activation (AMPK)

Triglycerides accumulation and AMPK activation by the polyphenolic compounds was evaluated by means of immunofluorescence with the 3T3-L1 hypertrophic insulin-resistant adipocyte model. On another hand, a double-blind placebo controlled clinical study with volunteers taking placebo or 500 mg a day of a dietetic supplement with Lemon Verbena and Rosella extracts in 65:35 weight ratio , respectively, was carried out, in which 56 overweight volunteers participated during two months. Anthropometric and biochemical parameters were determined.

3T3-L1 preadipocytes were cultured in a low glucose medium (1g/L) DMEM supplemented with 10 % bovine serum (CS), 100 µg/mL streptopmycin and 100 U/mL penicillin and were incubated at 37 °C in a 5 % volume CO₂ and a 95 % volume of air humidified atmosphere. Adipocyte differentiation was induced by means of a high glucose (4.5 g/L) medium culture supplemented with 10 foetal bovine serum (FBS), 1 µM insulin, 1 µM dexamethasone (DEX) and 0.5 mM de 3-isobutyl-1-methylxanthine (IBMX) during 48 hours. After 48 hours, the cells were kept in a high glucose medium with FBS and insulin, and the medium was replaced every two days, with which hypertrophic adipocytes were obtained after 20 days incubation. Once the hypertrophic adipocytes were obtained, the cells were treated with Lemon verbena extract (~ 30 % weight in phenylpropanoids) and Rosella extract (-10 % weight in anthocyanins) in a weight ratio 65 to 35, respectively, at different concentrations during 72 hours. The extracts were dissolved in medium and filtered in order to be sterilised.

The lipid content of the hypertrophic adipocytes was evaluated using the AdipoRed™ reactant. The cell supernatant was eliminated and these were carefully washed with phosphate buffered saline solution (PBS). Consecutively, AdipoRed™ was added and the cells were left to incubate during 15 minutes at ambient temperature. Triglyceride accumulation was measured using a microplate reader at 485 nm excitation wavelength and 572 nm emission wavelength.

For the sturdy of AMPK activation in hypertrophic adipocytes, phosphorylated AMPK in Thr172 was quantified (pAMPK) with an immunofluorescence assay. The cells were fixed with a fixation buffer, permeabilised with 0.3 % Triton x-100 (Sigma-Aldrich, Spain) and blocked with 4 % goat serum. Once this was done, the cells were incubated overnight at 4 °C with Alpha 1 + Alpha 2 mice AMPK monoclonal antibodies (Abcam, Cambridge, UK) and with rabbit Alpha 1 phospho-AMPK (Thr172; Cell Signalling Technology, Danvers, MA, USA). After the incubation with the primary antibodies, the cells were washed with PBS and incubated during 6 hours at ambient temperature with each corresponding secondary polyclonal antibody, goat anti-rabbit IgG CF™ 594 and anti-mouse FITC (both from Sigma-Aldrich, St. Louis, MO, USA). The cell fluorescence measured using a multimodal microplate reader with cell image capture (Cytation 3, Biotek, Spain) at 593 nm excitation wavelength and 614 nm emission wavelength to measure the AMPK levels. The AMPK activation is expressed as the ration between normalised AMPK and total AMPK. The results are shown in Figure 13. The p values are *p<0,05, and ***p<0,001.

The randomised, double-blind, placebo-controlled human study lasted 8 weeks. The exclusion criteria were a total cholesterol level lower than 200 mg/dL, the presence of any obesity-related pathology, the use of cholesterol or hypertension medication, the consumption of antioxidant supplements of pharmaceuticals, alcohol addiction and breastfeeding or pregnant women. Based on these criteria, 55 healthy women, aged between 36 and 69 years, with a body mass index between 25 to 34 kg/m², which passed a telephonic selection and an interview about their health as well as a biochemical and anthropometric assessment.

Once enrolled to the study, the volunteer were randomly assigned to the placebo group (n= 26) or to the experimental group (n=29). During the study, 9 volunteers quitted, 6 in the placebo group and 3 in the experimental group, and a total of 46 volunteers completed the study. The placebo group (mean age 51 years) received two placebo capsules, each containing 400 mg microcrystalline cellulose and the experimental group (mean age 52 years) received two capsules, each containing 250 mg of the Lemon Verbena and Rosella extracts composition and 150 mg excipients (microcrystalline cellulose). Therefore, the experimental group was taking 500 mg a day of the composition under study. Both capsule types had the same colour, colour, smell, and weight. The volunteers took two capsules a day 20 to 30 minutes before breakfast for two months. During the selection visit, demographic and lifestyle (age, diet, physical exercise routines, alcohol and tobacco consumption) information was gathered. A doctor asked the volunteers to follow an isocaloric diet with normal hydration and to walk at least 30 minutes a day. The study requirements fulfilment was confirmed in each visit and telephonically, each week, during the two months of study. The parameter measurements were taken at the start of the study and after 30 and 60 days of study.

During the study, several parameters were monitored. Anthropometric measurements were taken at the start of the study, after a month and after two months since the start of the study, consisting of body weight, height, skinfold measurements at the triceps, biceps and abdomen and arm and abdomen perimetric measurements. The abdomen perimeter (AC) was measured in two different places: frontally midways between the xiphoid process and the belly button and laterally midways between the thoracic chest end and the iliac crests (AC1) and at the belly button (AC2). The body fat percentage was obtained from the abdominal measurement using the Weltman equation.

Fasted blood samples were taken in order to measure total glucose, glycosylated haemoglobin and the lipids profile, which includes triglycerides, total cholesterol, good cholesterol (high-density lipoprotein) or HDL and bad cholesterol (low density lipoprotein) or LDL. The blood samples were analysed to measure security parameters such as haematology, electrolytes, creatinine, urea, uric acid, glutamic-pyruvic transaminase, glutamic-oxaloacetic transaminase and C-reactive protein.

Moreover, the heart rate at rest and diastolic and systolic tension were measured at the start, and after one and two months of study.

**Table 4. Anthropometric measurements, at the start and after one and two months of study. The intra-group statistical values at the end of the study compared with the initial values are: * p < 0,05; ** p < 0,01; *** p < 0,001; **** p < 0,0001. Data are expressed as mean values ± SD.**

| | **Experimental group (N= 26)** | | | **Placebo group(N= 20)** | | |
|---|---|---|---|---|---|---|
| | **Start** | **Month 1** | **Month** | **Start** | **Month 1** | **Month 2** |
| **Anthropometric parameters** | | | | | | |
| Body weight (kg) | 75.12 + | 72.76 ± 1351*** | 71.08 ± 1334*** | 75.16 ± 11.34 | 73.36 ± 11 24*** | 73.12 ± 11.47** |
| Body mass index | 29.20 ±4.76 | 28.26 ± 4.63*** | 27.59 ± 4.44*** | 30.23 ± 4.41 | 29.52 ± 4.42*** | 29.43 ± 4.58** |
| AC1(cm) | 90.87 ± | 86.59 ± 9.51*** | 84.39 ± 9.91*** | 90.93 ± 13.12 | 89.21 ± 12.83*** | 88.50 ± 11.65** |
| AC2 (cm) | 100.52 ±9.10 | 97.10 ± 8.20*** | 94.71 ± 8.94*** | 102.32 ± 11.59 | 99.87 ± 11.96** | 97.05 ± 10.66*** |
| Triceps skin fold | 28.36 ±7.57 | 26.08 ± 6.95*** | 24.67 ± 7.42*** | 30.13 ± 5.24 | 28.45 ± 4.75** | 27.88 ± 4.69*** |
| % body fat | 44.36 ±2.86 | 43.53 ± 2.73*** | 43.04 ± 2.64*** | 44.67 ± 2.82 | 44.18 ± 2.83*** | 43.95 ± 2.73*** |

| **Vital signs** | | | | | | |
|---|---|---|---|---|---|---|
| Heart rate (bpm) | 79.43 ±984 | 74.36 ± 9 03*** | 71 ± 8.40*** | 75.75 ± 8.62 | 75.15 ± 11.86 | 75.50 ± 11.61 |
| Sistolic tension mm Hg | 128.70 ± 12.74 | 118.43 ± 13.91*** | 109.96 ± 9.09*** | 126.65 ± 19.06 | 120.70 ± 17.50 | 118.05 ± 15.64* |
| Diastolic tension mm Hg | 77.62 ± 10.99 | 72.76 ± 10.54* | 67.05 ± 9.30*** | 78.30 ± 13.12 | 76.10 ± 8.16 | 71.10 ± 11.61* |

| **Biochemical parameters** | | | | | | |
|---|---|---|---|---|---|---|
| Glucose mg/dl | 93.33 ± 16.10 | 90.44 ± 14.11 | 90.06 ± 13.31 | 93.95 ± 12.96 | 94.05 ± 11.14 | 92.63 ± 14.95 |
| Tryglicerides mg/dl | 84.83 ± 52.61 | 76.28 ± 35.22 | 83.17 ± 39.39 | 85.63 ± 42.37 | 78.53 ± 27.95 | 88.74 ± 39.23 |
| Total cholesterol mg/dl | 237.78 ± 26.51 | 215.22 ± 26.90*** | 207.94 ± 25.11 *** | 229.11 ± 26.13 | 205.53 ± 24.84*** | 207.53 ± 28.62 ** |
| HDL mg/dl | 60.59 ± 7.24 | 59.23 ± 7.15 | 58.95 ± 8.52 | 59.00 ± 7.54 | 56.95 ± 9.02* | 57.11 ± 8.58 * |
| LDL mg/dl | 158.52 ± 25.17 | 140.90 ± 23.39 | 127.43 ± 20.83 *** | 153.68 ± 24.88 | 132.89 ± 23.18*** | 132.95 ± 25.39 *** |
| Creatinine mg/dl | 0.75 ± 0.11 | 0.85 ± 0.14 | 0.81 ± 0.12 | 0.80 ± 0.14 | 0.91 ± 0.18 | 0.82 ± 0.12 |
| Urea mg/dl | 33.36 ± 7.96 | 32.09 ± 8.52 | 32.32 ± 9.15 | 32.21 ± 10.54 | 32.37 ± 7.77 | 31.58 ± 7.44 |
| Uric acid mg/dl | 4.66 ± 1.01 | 4.29 ± 1.09 | 4.55 ± 1.18 | 4.97 ± 1.53 | 4.29 ± 1.03 | 4.48 ± 0.96 |
| GPT U/I | 22.59 ± 7.89 | 21.59 ± 9.53 | 21 ± 8.83 | 21.21 ± 6.82 | 21.61 ± 9.62 | 20.05 ± 8.44 |
| GOT U/I | 21.00 ± 5.07 | 21.59 ± 5.66 | 21.27 ± 6.85 | 21.11 ± 4.53 | 21.11 ± 5.51 | 20.21 ± 5.02 |

As it is shown in Figure 13, the composition with Lemon Verbena and Rosella extracts causes a potent increase in the pAMPK/AMPK ratio in a dose-dependent way which is statistically significant in comparison with the increase observed in the control experiment at a 350 µg/mL dose, whereas at a dose of 500 µg/mL an increase of approximately 1.5 is observed.

The anthropometric parameters of both groups do not show noticeable differences at the start of the study. The study results show a significant enhancement in the experimental group, particularly in body weight, abdominal perimeter and body fat percentage (see Table 4).

The experimental group showed a greater body weight reduction tan the placebo group which is statistically significant with a p value < 0.01. Both abdominal perimeters AC1 and AC2 were reduced after the two months of study, but only the highest abdominal perimeter showed statistically significant differences with the placebo group after one and two months of study. Consequently with these data, the body fat percentage was reduced in both groups, but in the experimental group it was statistically significant more reduced than in the placebo group after two months of study, p <0.01. Besides, also statistically significant differences were observed in the triceps skinfold measurement (p<0.05) and in the body mass index (p<0.01).

### EXAMPLE 5. Method of production of a composition with Lemon Verbena and Rosella extracts according with an aspect of the invention.

The composition with Lemon Verbena and Rosella extracts is a mixture of two extracts with a very specific active compounds composition, i.e. at least 10 % in weight of anthocyanins in the Rosella extract and at least 30 % in weight of phenylpropanoids in the Lemon Verbena extract, which, once mixed in a weight proportion of 35:65, respectively, exhibit synergic activity, especially for the control of satiety.

The manufacturing method of the Lemon Verbena extract comprises a hydroalcoholic extraction using alcohol concentrations (ethanol, methanol, propanol, isopropanol or butanol) greater than 70 and up to 100 % alcohol in volume in water (Figure 14).

The Lemon Verbena (*Lippia citriodora*) leafs are analysed in order to determine the amount of verbascoside using high-pressure liquid chromatography (HPLC). Approximately 330 g. of Lemon Verbena leafs are extracted in 4 litres of demineralised water-diluted ethanol (greater than 70 and up to 100 % of ethanol in volume) keeping the liquid stirred in the recipient, recirculating the evaporated solvent or percolating it in a *nutcha* filter at a temperature between 40 a 80 °C during two hours. After the two hours, the solution is filtered to separate the leaves and other particles. The filtered solution is vacuum-distilled at a temperature between 60 a 80 °C in order to obtain a concentrated aqueous solution which contains around 15 to 30 % in solids. This concentrated solution is spray-dried at a temperature of 70-90 °C in order to obtain a dry-powder extract that contains no less than 30 % in weight of total phenylpropanoids. The yield is from 19 to 21 in weight.

In order to produce the Rosella extract, the plant flowers are selected and analysed to obtain their anthocyanin content with high-pressure liquid chromatography (HPLC) and ultraviolet light detection. 320 g. of flowers are extracted with 3.2 litres of demineralised water-diluted ethanol at 50 % volume, keeping the liquid under stirring in the reactor, and recirculating the evaporated solvent or percolating it in a *nutcha* filter at a temperature between 40 y 60 °C during 2 hours. After the two hours extraction, the solution is filtered with a 0.5 micron pore filter to separate the flowers. The filtered solution is vacuum-distilled at a temperature between 50 y 70 °C and the obtained concentrate is diluted until obtaining 620 litres.

The product is subjected to a chromatographic purification step in a polymeric adsorption/desorption resin suitable for polyphenols. The eluate constitutes the purified extract in liquid form. The purified liquid is concentrates until 30 to 50 % in weight of solids. The concentrated dissolution is spray-dried at a temperature between 70 y 80 °C to obtain a dry-powder extract which contains no less than 10 % in weight of anthocyanins. The yield is from 5 to 7 % in weight.

After producing the Lemon Verbena and Rosella extracts separately, both are sieved and mixed and passed through a metal-detecting system before being packaged, as it is schematically shown in Figure 15.

## Claims

1. Composition for use in an individual's food intake modulation by appetite reduction and/or satiety increase through the individual's adipose tissue or digestive tract-secreted appetite-related peptides levels modification, **characterised in that** said composition comprises an extract with at least 5% in weight of anthocyanins and an extract with at least 15% in weight of phenylpropanoids, wherein the anthocyanins extract comprises a minimum of 25 % weight of Rosella (*Hibiscus sabdariffa*) extract and wherein the phenylpropanoids extract comprises a minimum of 50 % in weight of Lemon Verbena (*Lippia citriodora*) extract.

2. Composition according to claim 1 wherein the extract with at least 5% in weight of anthocyanins comprises an extract with around 10% in weight of anthocyanins.

3. Composition according to claim 1 or 2 wherein the extract with at least 15% in weight of phenylpropanoids comprises an extract with around 30% in weight of phenylpropanoids.

4. Composition according any preceding claim comprising around 65 % in weight of Lemon Verbena (*Lippia citriodora*) extract and around 35 % weight of Rosella *(Hibiscus sabdariffa)* extract.

5. Composition according to claim 4 administered in a daily dose of 250 to 1000 milligrams.

6. Composition according to claim 5 administered in a daily dose of 500 milligrams.

7. Composition for use in an individual's weight reduction by lipid metabolism modification mediated by the individual's AMP activated protein kinase energetic (AMPK) sensors which translates into the individual's body fat percentage reduction, **characterised in that** said composition comprises an extract with at least 5% in weight of anthocyanins and an extract with at least 15% in weight of phenylpropanoids, wherein the anthocyanins extract comprises a minimum of 25 % weight of Rosella (*Hibiscus sabdariffa*) extract and wherein the phenylpropanoids extract comprises a minimum of 50 % in weight of Lemon Verbena (*Lippia citriodora*) extract.

8. Composition according to claim 7 wherein the extract with at least 5% in weight of anthocyanins comprises an extract with around 10% in weight of anthocyanins.

9. Composition according to claim 7 or 8 wherein the extract with at least 15% in weight of phenylpropanoids comprises an extract with around 30% in weight of phenylpropanoids.

10. Composition according any claim from 7 to 9 comprising around 65 % in weight of Lemon Verbena (*Lippia citriodora*) extract and around 35 % weight of Rosella *(Hibiscus sabdariffa)* extract.

11. Composition according to claim 10 administered in a daily dose of 250 to 1000 milligrams.

12. Composition according to claim 11 administered in a daily dose of 500 milligrams.

13. Method of production of a composition that comprises an extract with at least 5% in weight of anthocyanins and an extract with at least 15% in weight of phenylpropanoids **characterised by** comprising a Lemon Verbena (*Lippia citriodora*) hydroalcoholic extraction and a Rosella (*Hibiscus sabdariffa*) hydroalcoholic extraction wherein the Lemon Verbena (*Lippia citriodora*) hydroalcoholic extraction is carried out using a alcohol in water dissolution with an alcohol content greater than 70 % and up to 100% in volume during two hours at a temperature between 40 and 80 °C.

14. Method according to claim 13 wherein the alcohol content is around 70 % in volume.
